(19) **Europäisches Patentamt European Patent Office Office européen des brevets**

(11) **EP 4 408 517 B1**

(12) **EUROPEAN PATENT SPECIFICATION**

(45) Date of publication and mention of the grant of the patent:
**05.08.2026 Bulletin 2026/32**

(21) Application number: **22875317.4**

(22) Date of filing: **21.09.2022**

(51) International Patent Classification (IPC):
*A61N 1/36* (2006.01)     *A61N 1/05* (2006.01)
*A61B 18/00* (2006.01)     *H02N 2/18* (2006.01)
*H02N 1/04* (2006.01)     *H10N 30/30* (2023.01)
*A61B 90/00* (2016.01)     *H10N 15/10* (2023.01)

(52) Cooperative Patent Classification (CPC):
**A61N 1/0504; A61N 1/0551; A61N 1/36057;**
**H02N 1/04; H02N 2/18; H10N 30/302;**
A61B 2090/065; H10N 15/15

(86) International application number:
**PCT/IL2022/051009**

(87) International publication number:
**WO 2023/053110 (06.04.2023 Gazette 2023/14)**

(54) **DEVICE AND SYSTEM FOR RESTORING TACTILE SENSATION USING A NANOGENERATOR**

VORRICHTUNG UND SYSTEM ZUR WIEDERHERSTELLUNG EINER TASTEMPFINDUNG UNTER
VERWENDUNG EINES NANOGENERATORS

DISPOSITIF ET SYSTÈME DE RESTAURATION DE SENSATION TACTILE À L'AIDE D'UN
NANOGÉNÉRATEUR

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.09.2021 US 202163249345 P**

(43) Date of publication of application:
**07.08.2024 Bulletin 2024/32**

(73) Proprietors:
• **Ramot at Tel-Aviv University Ltd.**
  **Tel-Aviv 6139201 (IL)**
• **Sheba Impact Ltd.**
  **5265601 Ramat-Gan (IL)**

(72) Inventors:
• **MAOZ, Ben Meir**
  **6139201 Tel Aviv (IL)**
• **MARKOVICH, Gil**
  **6139201 Tel Aviv (IL)**
• **ARAMI, Amir**
  **5265601 Ramat Gan (IL)**
• **SHLOMY, Iftach**
  **6139201 Tel Aviv (IL)**

(74) Representative: **Boult Wade Tennant LLP**
**Salisbury Square House**
**8 Salisbury Square**
**London EC4Y 8AP (GB)**

(56) References cited:
CN-A- 111 408 046     CN-A- 111 408 046
US-A1- 2015 076 964     US-B2- 10 369 362

• SHLOMY IFTACH ET AL: "Restoring Tactile
Sensation Using a Triboelectric Nanogenerator",
vol. 15, no. 7, 17 June 2021 (2021-06-17), US,
pages 11087 - 11098, XP093054535, ISSN:
1936-0851, Retrieved from the Internet
<URL:https://pubs.acs.org/doi/pdf/10.1021/
acsnano.0c10141> [retrieved on 20230119], DOI:
10.1021/acsnano.0c10141
• SHLOMY IFTACH, DIVALD SHAY, TADMOR
KESHET, LEICHTMANN-BARDOOGO YAEL,
ARAMI AMIR, MAOZ BEN M.: "Restoring Tactile
Sensation Using a Triboelectric Nanogenerator",
ACS NANO, vol. 15, no. 7, 27 July 2021
(2021-07-27), US
, pages 11087 - 11098, XP093054535, ISSN:
1936-0851, DOI: 10.1021/acsnano.0c10141

• KORKMAZ SATIYE ET AL.: "Production and applications of flexible/wearable triboelectric nanogenerator (TENGS)", SYNTHETIC METALS, vol. 273, 5 January 2021 (2021-01-05), CH , XP086500614, ISSN: 0379-6779, DOI: 10.1016/ j.synthmet.2020.116692

## Description

## TECHNICAL FIELD

The present invention relates to devices and systems for restoring tactile sensation to subjects in need thereof, and more particularly, to a device and system for restoring tactile sensation using an implanted nanogenerator.

## BACKGROUND

Traumatic peripheral nerve injury (TPNI) is a common disorder that affects 2.8% of trauma patients, and can result in lifelong disability, chronic pain, and diminished quality of life. A common effect of TPNI is a loss of tactile sensation, which not only interferes with patients' daily lives but also increases susceptibility to injury. Currently, few solutions are available for restoration of tactile sensation. The gold standard solution is surgical nerve reconstruction by nerve autograft, nerve conduits, or nerve allografts. Unfortunately, nerve reconstruction can only be performed in a limited time period (i.e., in the first two years after injury), and it requires healthy skin with viable end-organs. Moreover, even when these conditions are met, its success rate is low.

An alternative for the restoration of tactile sensation is the development of wearable or implanted neuro-prosthetic devices that simulate the experience of touch. This simulation is achieved by translating pressure cues around the damaged area into electrical signals that can subsequently be processed by the brain. Several such devices have been proposed and implemented, using various technologies, such as computer-brain interfaces and "electronic skin" that can mimic the sensory properties of skin and some of its biological properties (e.g., stretchability). Neuro-prosthetic technologies are still in early stage however, and only a few have undergone proof-of-principle testing in vivo. Moreover, the tools developed thus far have several key shortcomings: First, they are expensive and complex to implement, with some (e.g., electronic skin) requiring supplementary support platforms. These features suggest that the process of adapting current technologies into devices that are appropriate for widespread clinical use is likely to be prolonged, and, without significant advances in cost reduction, the finished product may still be inaccessible to many patients. Second, current neuro-prosthetic technologies require a power source, typically an external power source or a battery. Such power sources may be inconvenient to replace, and they might risk introducing toxic materials in the case of malfunction. Third, the neuro-prosthetic technologies that have been implemented in patients tend to require long periods of training and adjustment.

SHLOMY IFTACH ET AL. ("Restoring Tactile Sensation Using a Triboelectric Nanogenerator", ACS NANO, vol. 15, no. 7, 17 June 2021, pages 11087-11098) discloses an implantable triboelectric nanogenerator (TENG) device for restoring tactile sensation. The TENG-IT device is implanted under the skin and translates tactile pressure into electrical potential, which it relays via cuff electrodes to healthy sensory nerves, thereby stimulating them to mimic tactile sensation. The device is self-powered, biocompatible, sensitive, and flexible. The device comprises a small number of components and is constructed from affordable materials, with a straightforward fabrication process. The TENG-IT was demonstrated in vitro to elicit electrical activity in sensory neurons (DRGs) dependent on the level of pressure applied, and in vivo to provide tactile sensory capabilities to rodents in which a segment of a sensory nerve was removed.

US 2015/0076964 A1 (MADANI) discloses an apparatus for generating electric energy from a nanogenerator embedded in a thin tape. The apparatus comprises an array of nanogenerators with piezoelectric and/or thermoelectric materials embedded in a transparent tape that may be pasted on a floor in a crowded place. The nanogenerator comprises a substrate, a piezoelectric material (such as barium titanate, $BaTiO_3$), and a thermoelectric material (such as bismuth telluride, $Bi2Te_3$) arranged together with piezoelectric nanowires (ZnO). The apparatus generates an electric field by using variations in pressure and surface temperature, which is then transferred to a charging/discharging board in the form of electric energy for later use. The tape may have a graphene coating on the top end, which functions as an electrode and aids in better conductivity. The graphene coating has a thickness of 50 nm to 200 nm, preferably 100 nm, and is heat sensitive, changing its conductivity properties in response to temperature variations.

CN 111408046 A (BEIJING INST NANOENERGY & NANOSYSTEMS) discloses an electrical stimulation system for promoting nerve repair in vivo. The system comprises a nanogenerator, a wire, and an electrode, wherein the nanogenerator is connected to the electrode through the wire to transmit current or voltage generated by the nanogenerator to the electrode, and the electrode is capable of applying current or voltage to an organism. The system may be implantable or wearable. The nanogenerator may be a triboelectric nanogenerator and/or a piezoelectric nanogenerator. The triboelectric nanogenerator includes a core friction structure comprising a polymer friction layer, metal layers, and spacers. The electrode may be a needle electrode, spiral wire electrode, or interdigital electrode made of gold and/or platinum. The electrode may be encapsulated with a flexible insulating material such as polydimethylsiloxane (PDMS), epoxy resin, silicone rubber, or parylene. The nanogenerator may also include an encapsulation layer of flexible insulating material with a thickness of 5-800 micrometers. The system is self-powered and can be driven by body movement and force to generate electricity for stimulating nerve cells and tissues to promote repair.

Therefore, there is a need in the art for an implantable device for restoring tactile sensation, which overcomes the shortcoming of the art, by being cost effective, biocompatible, sensitive, having small scale factor, easy to implement and use and which does not necessarily require a power source.

## SUMMARY

The invention is defined by the independent claim 1. According to some embodiments, there are provided herein advantageous devices, systems and non-claimed methods for restoring tactile sensation to subjects in need thereof by utilizing an implantable device, which has pressure sensitivity and which is capable of relaying an electric signal is response to said pressure.

An aspect of some embodiments of the current disclosure relates to devices, systems and non-claimed methods for restoring tactile sensation using a nanogenerator (e.g., piezoelectric, triboelectric, and/or pyroelectric nanogenerators).

According to some embodiments, the devices, systems and non-claimed methods disclosed herein are advantageous as, they can provide at least some restoration of tactile sensation to a subject in need thereof in a sensitive and cost effective manner. As exemplified herein, the implantable device disclosed herein is biocompatible (i.e., made of and/or coated with a biocompatible material), to prevent damage to tissues surrounding the implanted region. Additionally, the device is flexible, durable, and has a small-factor size, rendering it particularly suitable for implementation at, for example, small areas (such as limb extremities, for example, fingers). Moreover, advantageously, the devices, systems and non-claimed methods disclosed herein can allow a wide range of sensitivity, essentially corresponding to normal human pressure perception, which can range from a few kPa for a gentle touch to tens of kPa for object manipulation. Furthermore, the device disclosed herein is readily implantable, using routine minimal surgical procedures, and use thereof by the implanted subject is easy and/or intuitive.

According to some embodiments there is provided an implantable device for at least partially restoring tactile sensation to a subject in need thereof, the device including:

at least one nanogenerator unit configured to be positioned subcutaneously and to produce an electric signal upon exertion of an external stimulus; and
at least one electrode configured to be connected to at least one functional sensory nerve of the subject;
such that the electric signal produced by the at least one nanogenerator unit in response to the external stimulus is conveyed to the at least one functional sensory nerve of the subject, via the at least one electrode, thereby at least partially restoring the tactile sensation to the subject.

According to some embodiments, the electric signal may be proportional to the magnitude of the external stimulus. Optionally, the external stimulus may be pressure, friction, traction, shear force, and/or temperature change (e.g., heating and/or cooling). Optionally, the electric signal may be voltage or current.

According to some embodiments, the at least one electrode may be a cuff electrode.

According to some embodiments, the sensory restoration device may include a plurality of nanogenerator units. Optionally, a single nanogenerator unit may be connected to a single nerve. Optionally, the plurality of nanogenerator units may be connected to a single nerve. Optionally, the sensory restoration device may include a plurality of nanogenerator units connected to a plurality of nerves. Optionally, the plurality of nanogenerator units may be connected in an array. Optionally, the array may include the same type of nanogenerator unit. Optionally, the array may include different types of nanogenerator units. Optionally, the array may include a mixture of nanogenerator units which may be the same and/or different.

According to some embodiments, the nanogenerator unit may be selected from the group consisting of a piezoelectric nanogenerator, a triboelectric nanogenerator, and a pyroelectric nanogenerator.

According to some embodiments, the pyroelectric nanogenerator may be a thermal sensor and/or thermal coating. Optionally, the response time and/or reset time of the pyroelectric nanogenerator may be more than 0.001 s.

According to some embodiments, the piezoelectric nanogenerator may have a wurtzite structure or a perovskite structure. Optionally, the piezoelectric nanogenerator may be a pressure sensor.

According to some embodiments, the triboelectric nanogenerator (TENG) may be configured to convert pressure, friction and/or traction force applied to said TENG into an electric signal that may be supplied to at least one sensory nerve. Optionally, the electric signal is proportional to said applied friction and/or traction force.

According to some embodiments, the sensory restoration device may include circuitry which may be configured to change the electrical features of an electric pulse generated by the TENG.

According to some embodiments, the TENG may be further coated with a heat sensitive material. Optionally, the nanogenerator is further coated with a heat sensitive material.

According to some embodiments, the nanogenerator may be self-powered. According to some embodiments, the device may be biocompatible.

According to some embodiments, a non-claimed method for at least partially restoring tactile sensation to a subject in need thereof, may include the steps of:

providing a sensory restoration device comprising at least one nanogenerator and an electrode to a subject in need thereof; wherein the device may be configured to generate an electric signal by the nanogenerator in response to an external stimulus of a body region which corresponds to a subcutaneous location at which the at least one nanogenerator is located; and may convey the generated electric signal from nanogenerator via the electrode to a functional nerve associated with the electrode. Optionally, wherein the subject may have an impaired tactile sensation caused by nerve damage.

According to some embodiments, the sensory restoration device may be subcutaneously implanted. According to some embodiments, the sensory restoration device may be electrically connected to the functional nerve of the subject via the electrode.

According to some embodiments, the external stimulus may include pressure, friction, traction, shear forces and/or temperature change (e.g., heating and/or cooling). Optionally, the electrical signal may be proportional to the magnitude of the external stimulus.

According to some embodiments, the device may be implanted such that the nanogenerator may be placed subcutaneously and/or the electrode is in physical contact with a corresponding functional nerve.

According to some embodiments, an external controller may be configured to control one or more operating parameters of the device. Optionally, the controller may be wirelessly associated with the device. Optionally, the controller may be configured to control the sensitivity of the sensory restoration device.

According to some embodiments, the controller may be configured to determine and/or provide feedback on the condition/operation of the sensory restoration device.

Certain embodiments of the present disclosure may include some, all, or none of the above advantages. One or more other technical advantages may be readily apparent to those skilled in the art from the figures, descriptions, and claims included herein. Moreover, while specific advantages have been enumerated above, various embodiments may include all, some, or none of the enumerated advantages.

## BRIEF DESCRIPTION OF THE FIGURES

Some embodiments of the disclosure are described herein with reference to the accompanying figures. The description, together with the figures, makes apparent to a person having ordinary skill in the art how some embodiments may be practiced. The figures are for the purpose of illustrative description and no attempt is made to show structural details of an embodiment in more detail than is necessary for a fundamental understanding of the disclosure. For the sake of clarity, some objects depicted in the figures are not to scale.

In the Figures:

**Figs. 1A-B:** Illustration of an exemplary TENG device, in accordance with some embodiments. **Fig. 1A** is a schematic diagram of an exemplary use of a TENG; **Fig. 1B** are photographs of an exemplary cuff electrode (top) and TENG device (bottom);

**Fig. 2:** Schematic illustration of an the TENG, which converts mechanical energy into electricity using the triboelectric effect, in accordance with some embodiments;

**Fig. 3A-C:** Schematic illustrations of the working modes of the TENG, in accordance with some embodiments. **Fig. 3A** is a schematic illustration of a vertical mode; **Fig. 3B** is a schematic illustration of a sliding mode; and **Fig 3C** is a schematic illustration of a single electrode mode;

**Fig. 4:** An illustration of an exemplary TENG array implanted in a human finger, in accordance with some embodiments;

**Fig. 5:** A schematic illustration of an exemplary manufacturing process for a TENG device, in accordance with some embodiments;

**Figs. 6A-C:** Various views **(Fig. 6A,** side view; **Fig. 6B,** perspective view; and **Fig. 6C,** expanded view) of a schematic illustration of an exemplary device design and structure of a TENG device, in accordance with some embodiments;

**Figs. 7A-E:** schematic diagrams of an exemplary design of a TENG array, in accordance with some embodiments. **Fig. 7A-** top view of a TENG array; **Fig. 7B-** Side view of spacers of an array; **Fig. 7C-** top view of first and second electrodes of a TENG array; **Fig. 7D-** a side view spacers; **Fig. 7E** shows a top view of first and second circles of electrodes of the TENG array;

**Fig. 8:** A top view of an exemplary template prepared by 3D printing, in accordance with some embodiments;

**Fig. 9:** A top view of an exemplary template prepared by 3D printing, in accordance with some embodiments;

**Fig. 10:** A perspective view of a schematic illustration of an exemplary TENG array template, in accordance with some embodiments;

**Fig. 11:** An exemplary image of a top view of a TENG array, in accordance with some embodiments;

**Fig. 12:** A graph showing the effect of Kapton thickness on the TENG device's response, in accordance with some embodiments;

**Fig. 13:** A graph showing average peak-to-peak output voltage from an encapsulated TENG device with different spacer heights, upon application of 1 kPa pressure. N=3 each, in accordance with some embodiments;

**Figs. 14A-D:** Exemplary TENG device characterization, in accordance with some embodiments. **Fig. 14A** shows a schematic of the TENG layers (left) and a photograph of an exemplary TENG (right). **Fig. 14B** shows mean peak-to-peak electrical output modulation of TENG as a function of the materials used for the friction layers. **Fig. 14C** shows output performance of TENG for different levels of pressure applied. **Fig. 14D** shows TENG response to physiological pressure;

**Figs. 15A-E:** Exemplary TENG device characterization, in accordance with some embodiments. **Fig. 15A** Average peak-to-peak output voltage from TENG device. Insets in show raw data corresponding to 5 s intervals after specific time points. **Fig. 15B** SEM images of the TENG device. **Fig. 15C** Average peak-to-peak output voltage of the TENG device in response to a set of increasing and decreasing accelerations (pressure). **Fig. 15D** shows Example of TENG device compression. **Fig. 15E-** Average peak-to-peak output voltage divided by the initial peak-to-peak measurement;

**Fig. 16:** A graph showing an exemplary change in the electrical features of the electric pulse generated by the TENG, and exemplary circuitry of the array, in accordance with some embodiments;

**Figs.17A-C:** *In vitro* testing of an exemplary TENG, in accordance with some embodiments. **Fig. 17A** is a $\times 10$ image of an exemplary DRG on MEA culture at DIV 5, with complete axonal coverage. **Fig. 17B-** Raw MEA data, with the threshold for spike definition marked as a horizontal line, and vertical lines generating the raster plot of activity. KCl was added to reach 50 mM concentration at $t = 10$ s. **Fig. 17C-** Data analysis for 4 representative electrodes: Raster plot, rate histogram of spikes per 0.1 seconds, and average waveform shape for each spike; and

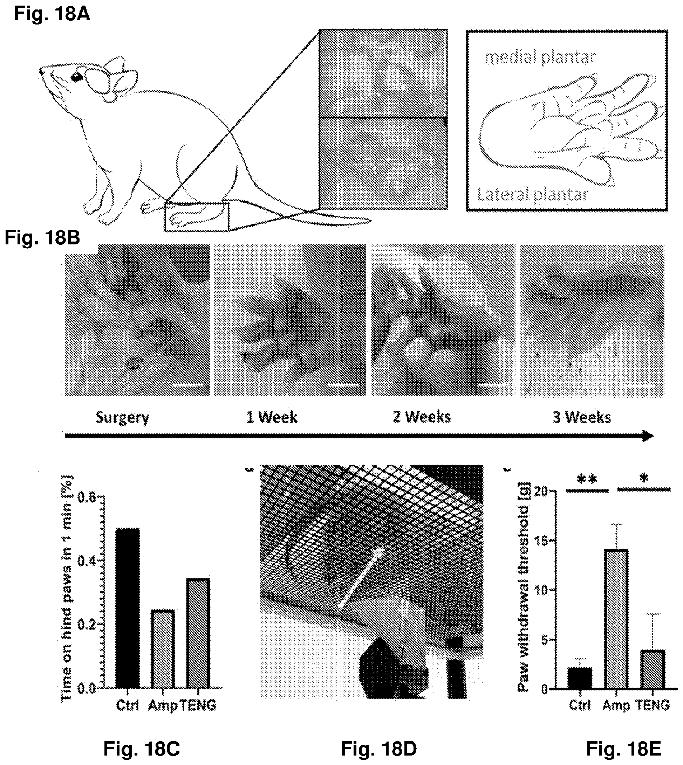

**Figs. 18A-E:** *In vivo* testing of an exemplary TENG, in accordance with some embodiments. **Fig. 18A-** Schematic illustration based on anatomical dissection of the nerves of a Wistar rat's hindfoot. **Fig. 18B** Images of surgical implantation of TENG device (8 mm $\times$ 3 mm) in a rat's left hindfoot, and post-operative recovery (scale = 1 cm). **Fig. 18C.** Percentage of time spent on hind paws for each group, over the course of 1 min. **Fig. 18D** shows an exemplary von Frey test setup. Arrow points to the tip of the device. **Fig. 18E-** Difference in average required peak force between the right and left hind paws in the von Frey test, measured for three experimental groups: control, amputee, TENG device.

## DETAILED DESCRIPTION

The principles, uses and implementations of the teachings herein may be better understood with reference to the accompanying description and figures. Upon perusal of the description and figures present herein, one skilled in the art will be able to implement the teachings herein without undue effort or experimentation. In the figures, same reference numerals refer to same parts throughout.

In the following description, various aspects of the invention will be described. For the purpose of explanation, specific details are set forth in order to provide a thorough understanding of the invention. However, it will also be apparent to one skilled in the art that the invention may be practiced without specific details being presented herein. Furthermore, well-known features may be omitted or simplified in order not to obscure the invention.

In the following description, numerous details are set forth for the purpose of explanation. However, one of ordinary skill in the art will realize that the invention may be practiced without the use of these specific details.

According to some embodiments, there are provided herein devices, systems and non-claimed methods for restoring tactile sensation using an implantable nanogenerator (e.g., piezoelectric, triboelectric, and/or pyroelectric nanogenerators) to a subject in need thereof.

According to some embodiments, as used herein, a "nanogenerator" may convert mechanical and/or thermal energy, such as produced by a small-scale physical change, into electricity. Optionally, a nanogenerator may be a piezoelectric, triboelectric, and/or pyroelectric nanogenerator. Optionally, a nanogenerator may convert energy from an external stimulus into electricity. Optionally, the external stimulus may be a change in pressure and/or temperature. Optionally, both piezoelectric and triboelectric nanogenerators may convert mechanical energy into electricity. Optionally, a piezoelectric nanogenerator may have a permanent electrostatic charge. Optionally, a triboelectric nanogenerator may have a temporary electrostatic charge. Optionally, a pyroelectric nanogenerator may harvest thermal energy from a temperature fluctuation. In some exemplary embodiments, the nanogenerator is a triboelectric nanogenerator (TENG).

As used herein, the term "a subject in need thereof" relates to a subject having an impaired tactile sensation due to nerve damage or malfunction.

As used herein, the term "tactile sensation" relates to sense of touch where contact, pressure or traction exerted on the skin (or other organs) of the subject. In some embodiments, the term tactile sensation also relates to the sensation of temperature (heat).

As used herein, the term "subcutaneous" relates to beneath, or under, all the layers of the skin.

As used herein, the term "external stimulus" relates to a stimulating action which is exerted on an outer surface (such as, skin) which is capable (directly or indirectly) to be sensed and/or to affect an internal (e.g., an implanted, subcutaneously located) sensor/nanogenerator device. Such external stimuli may include, for example, application of force, pressure, traction, friction, temperature changes, and the like. In some embodiments, the external stimulus may be translated to/generate/induce an electric signal by the nanogenerator. In some embodiments, the external stimulus is topical. In some embodiments, the external stimulus is at a region/area which is external (for example, topical) to the corresponding internal (implanted) region/area in which the nanogenerator device is located

As used herein, the term "desensitized" relates to nerve damage resulting in complete or partial lack of sensation, for example, by neurapraxia, axonotmesis, or neurotmesis.

As used herein, the term "healthy", "healthy tissue", "functional" and "healthy nerve" relates to at least partially functional, undamaged tissue. According to some embodiments, the sensory restoration device may be used to convert an external stimulus (such as mechanical (e.g., pressure, traction, etc.) and optionally thermal energy) into electricity, e.g., triboelectrification, electrostatic induction, etc., which may be conducted/conveyed to a corresponding healthy nerve, for example, the closest healthy (e.g., functional) nerve. Optionally, the amount of electricity and/or the magnitude of the electric signal may be proportional to the size of the external stimulus, e.g., application of higher pressure may produce more electricity than application of a low pressure. Likewise, a large temperature change may produce more electricity that a small temperature change.

According to some embodiments, the sensory restoration device may advantageously comprise a small number of components and/or may be constructed from affordable and biocompatible materials using a straightforward fabrication process. Advantageously, the sensory restoration device may provide an affordable, accessible, self-powered, and sensitive device for restoring tactile sensation. Advantageously, the sensory restoration device may be self-powered and/or biocompatible.

According to some embodiments, a sensory restoration device may be composed of several different layers with different functions. Optionally, the sensory restoration device may comprise an array of nanogenerator units. Optionally, the array may include at least one type of nanogenerator units. Optionally, the type of nanogenerator unit may be selected from the group consisting of a piezoelectric nanogenerator, a triboelectric nanogenerator, and a pyroelectric nanogenerator. Each possibility is a separate embodiment. Optionally, the sensory restoration device may include a signal changer. Optionally, the signal changer may modify one or more signals received from one or more nanogenerators. Optionally, the signal changer may filter out low intensity and/or background signals.

According to some embodiments, a sensory restoration device may comprise a triboelectric nanogenerator (TENG). Optionally, the TENG may be a self-powered device that may be implanted under the skin (e.g., at the fingertip) and may transform touch into voltage, which may be transduced to healthy sensory nerves e.g., via cuff electrodes, and may excite peripheral nerves proportionally to the pressure that is applied on the device.

According to some embodiments, the sensory restoration device may include a piezoelectric nanogenerator. Optionally, the piezoelectric material may be crystalline, ceramic, or polymeric. Optionally, the piezoelectric nanogenerator may include one or more piezoelectric compounds. Optionally, the piezoelectric compound may have a wurtzite structure or a perovskite structure. Optionally, the piezoelectric compound may be ZnO (n-type), ZnO (p-type), ZnO-ZnS. GaN, CdS, $BaTiO_3$, polyvinylidene fluoride (PVDF), $KNbO_3$, lead magnesium niobate-lead titanate (PMN-PT), lead zirconate titanate (PZT), barium titanate, lead titanate, gallium nitride, zinc oxide, quartz, Rochelle salt, topaz, tourmaline-group minerals, silk, wood, enamel, dentin, potassium niobate, lithium niobate, lithium tantalate, any other compound having piezoelectric properties, and/or combinations thereof. Optionally, the piezoelectric compound may be a pressure sensor.

According to some embodiments, the sensory restoration device may include a pyroelectric nanogenerator. Optionally, the sensory restoration device may include a heat sensitive compound, thermal sensor and/or thermal coating, e.g., using a material which may change its conductivity due to heating, such as $VO_2$ (vanadium dioxide) which is an insulator at room temp but conductive at 50°C. Optionally, the pyroelectric material may be boron aluminum nitride (BAlN) and boron gallium nitride (BGaN), tourmaline, gallium nitride, cesium nitrate ($CsNO_3$), polyvinyl fluoride, derivatives of phenyl pyridine, cobalt phthalocyanine, lithium tantalite ($LiTaO_3$) any other compound having pyroelectric properties, and/or combinations thereof.

According to some embodiments, the pyroelectric nanogenerator may have a response time of more than about 0.001 s, more than about 0.005 s, more than about 0.01 s, more than about 0.05 s, more than about 0.1 s, more than about 0.5 s, more than about 1 s, or more than about 5 s. Optionally, the pyroelectric nanogenerator may have a reset time of more than about 0.001 s, more than about 0.005 s, more than about 0.01 s, more than about 0.05 s, more than about 0.1 s, more than about 0.5 s, more than about 1 s, or more than about 5 s.

According to some exemplary embodiments, the sensory restoration device may relate to non-harmful mechanical stimulations associated with simple touch perception and/or object manipulation. According to some embodiments, the sensory restoration device may be used for restoring tactile sensation. According to some embodiments, the sensory restoration device may advantageously be a stable, sensitive to pressure along the physiological range, durable, biocompatible, and capable of generating a large triboelectric effect.

According to some embodiments, the sensory restoration device may include an interface with the nervous system. Optionally, the integration point may be located at the central nervous system (CNS) and/or peripheral nervous system (PNS) level. Optionally, the desired use of the device may define design criteria and/or properties of neural integration method, such as stability and cell specificity.

According to some embodiments, delivery of stimulation from the sensory restoration device to the nervous system may be achieved in numerous ways, such as electrodes, optics, acoustic and electromagnetic inductions. Optionally, the sensory restoration device may include stimulating electrodes and/or a mechanism of integrating an artificial signal transducer with the peripheral nerve system. Optionally, transmitting electrical current into a viable nerve may include a surface electrode (e.g., cuff electrode), penetrating electrodes (LIFE and TIME) and/or regenerative electrodes. Optionally, stability may be affected by the electrode design, the implantation method and the bonds created between the implant and the nervous tissue. Optionally, selectivity may be a measure of the stimulating electrode spatial resolution and its ability to reach the exact nerve, fascicle or axon of target. Optionally, the electrode may be a Cuff electrode to relay the sensory restoration device generated potential to one or more healthy (i.e., functional, undamaged) nerves. Optionally, at least one nanogenerator unit may be configured to be implanted directly on the tip of a healthy nerve without the need for dedicated electrodes. Optionally, each nanogenerator unit may be connected to a single nerve. Optionally, a plurality of nanogenerator units may be connected to a single nerve. Optionally, a plurality of nanogenerator units may be connected to a plurality of nerves.

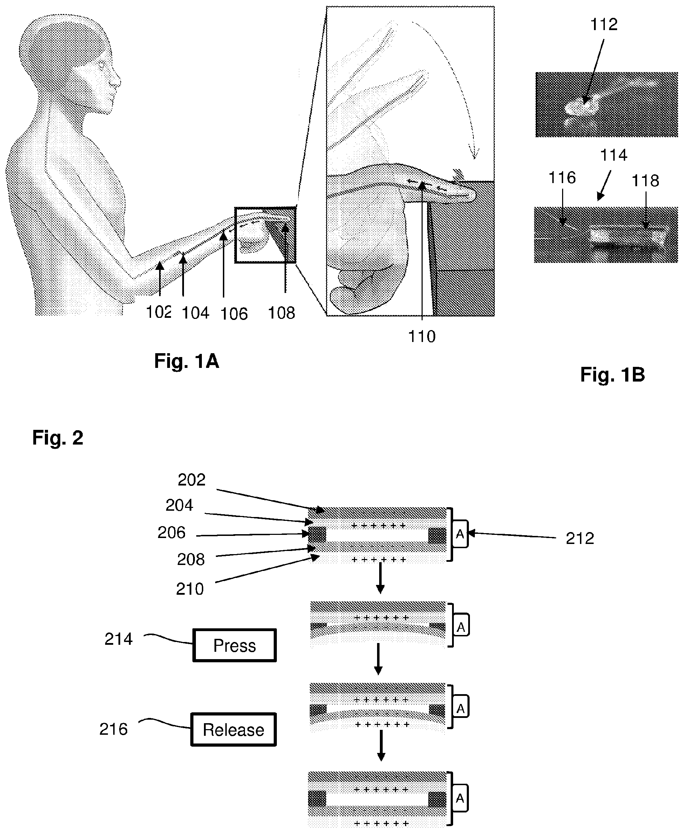

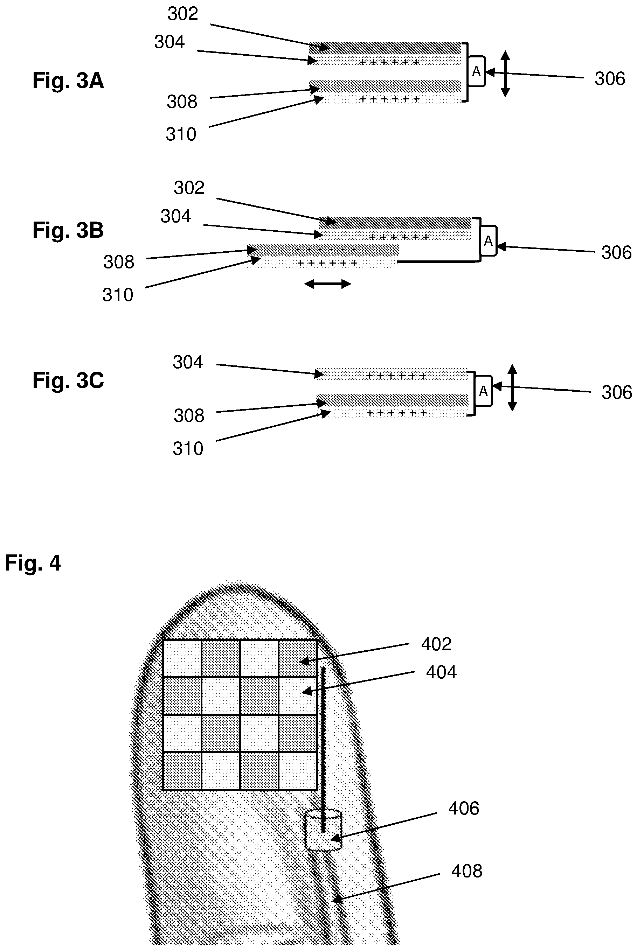

According to some embodiments, the sensory restoration device may include a nanogenerator unit. Optionally, the nanogenerator unit may be a TENG. Optionally, the TENG may include a first electrode including a metal, connected to a positive triboelectric material, one or more spacers, a negative triboelectric material, and a second electrode. According to some embodiments, contact between the positive and negative triboelectric materials may result in contact electrification, thereby providing static polarized charges on the material surface, and/or electrostatic induction, thereby turning the mechanically triggered change in potential into current (e.g., **Fig. 1B).** Optionally, two triboelectric materials may come into contact by application of an external force, e.g., pressing, bending, sliding, etc. (e.g., **Figs. 3A-C).** Optionally, induction of triboelectric charge may occur on the surface of the triboelectric materials. Optionally, once the external force is released, the two charged surfaces of the triboelectric materials may be separated, and the current may stop. Optionally, both triboelectric materials may be connected to a back electrode on the opposite side of the contact. Optionally, a potential difference may be built upon these electrodes. Optionally, the connection between the electrodes to an external load may allow current to flow between them. Optionally, this may screen out the electric field. Optionally, the external force may be applied repeatedly. Optionally, each application of an external force may bring the two surfaces back into contact. Optionally, the potential difference may change on each repetition. Optionally, the direction of current may alternate on each contact.

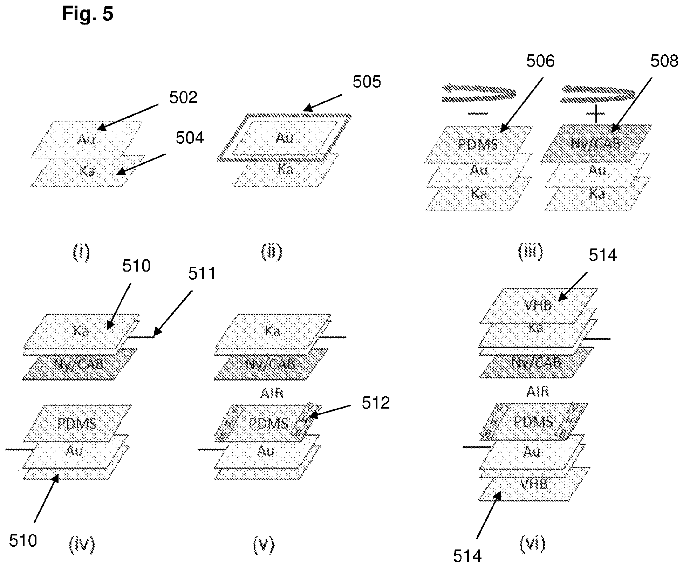

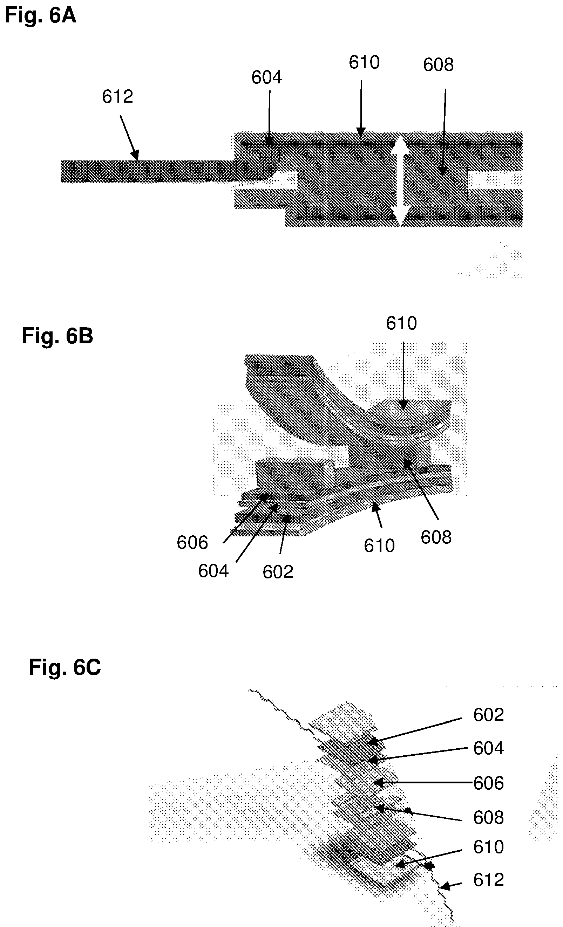

According to some embodiments, a TENG may include several layers (e.g., **Fig. 5 and Figs. 6A-C).** Optionally, the innermost layer may be the friction layer that creates the triboelectric effect, which may allow the device to be self-powered. Optionally, these layers may be separated by spacers. Optionally, the spacers may be thin, flexible strips. Optionally, the spacers may allow contact and separation of the friction layers, which may produce the triboelectric charge. Optionally, each of the friction layers may be attached to a conductive film. Optionally, the conductive film may function as an electrode, which may facilitate charge transfer. Optionally, the device may be encapsulated in a biocompatible isolating material (e.g., polydimethylsiloxane and/or fibrin glue). Optionally, the encapsulating layer may prevent contact between the TENG device and the surrounding physiological environment.

According to some embodiments, a sensory restoration device may be of various sizes and shapes. Optionally, a sensory restoration device may be circular, rectangular, square, octagonal, hexagonal, pentagonal, triangular, etc. Optionally, the sensory restoration device may include one or more nanogenerator unit in an array. Optionally, the nanogenerator units in an array may be set out as a grid, spiral, cluster, radiating star, circular, etc. Optionally, a nanogenerator unit may be fabricated as sheets and cut to the appropriate size and/or shape. Optionally, a template for a nanogenerator unit and/or array may be prepared by 3D printing. Optionally, the template may be filled with a dielectric material. Optionally, the dielectric material may be a positive dielectric material, such as nylon (Ny), cellulose acetate butyrate (CAB), etc., and/or a negative dielectric material, such as polydimethylsiloxane (PDMS), etc.

According to some embodiments, a sensory restoration device may be an array which may include nanogenerator units of different types, sizes and/or with different levels of sensitivity for implantation in different parts of the body. Optionally, an array may include between about 1 to about 1,000, between about 1 to about 500, between about 1 to about 100, between about 1 to about 50, or between about 1 to about 10 nanogenerator devices.

According to some embodiments, area of each sensory restoration device may range in size between about 0.05 $mm^2$ to about 0.1 $mm^2$, between about 0.1 $mm^2$ to about 0.5 $mm^2$, between about 0.5 $mm^2$ to about 1 $mm^2$, between about 1 $mm^2$ to about 5 $mm^2$, between about 5 $mm^2$ to about 10 $mm^2$, between about 10 $mm^2$ to about 25 $mm^2$, between about 25 $mm^2$ to about 50 $mm^2$, between about 50 $mm^2$ to about 100 $mm^2$, or between about 100 $mm^2$ to about 250 $mm^2$.

According to some embodiments, a circular TENG array may include alternating positive and negative dielectric material. Optionally, a circular TENG array may include one or more spacers. Optionally, the one or more spacers may be circular. Optionally, the one or more spacers may be linear. Optionally, the one or more spacers may be by radial. Optionally, the one or more spacers may include one or more holes. Optionally, one or more wires may pass through the one or more holes. Optionally, the one or more wires may be affixed to the positive and/or negative dielectric material. Optionally, a circular TENG array may include one or more leaves. Optionally, may include a first electrode and a second electrode. Optionally, the one or more wires may be affixed to a first electrode and a second electrode. Optionally, a circular TENG may be sensitive to shear (e.g., lateral forces) and/or pressure (e.g., vertical forces).

According to some embodiments, a TENG device may be include a positive and negative dielectric material, which may serve as an electrode (e.g., **Fig. 1**). Optionally, the dielectric may be biocompatible. Optionally, the dielectric may be flexible. Optionally, the dielectric may have the potential to generate large electrical potential.

According to some embodiments, a positive dielectric material may be selected from: nylon (Ny), cellulose acetate butyrate (CAB), acetate, silk, steel, wool, cellulose based polymers, polyamides, polyethylene glycol based polymers, polyvinyl alcohol, polymethyl methacrylate polymers, etc. Each possibility is a separate embodiment.

According to some embodiments, a negative dielectric materials may be selected from the group including polydimethylsiloxane (PDMS), polyvinyl chloride, polyalkylene, polytetrafluoroethylene, rubber, polyester, polyurethane, polystyrene, polychloroethane, etc. Each possibility is a separate embodiment.

According to some embodiments, a TENG device may be include a metal. Optionally, the metal may act as the electrode. Optionally, the metal may be selected from the group including gold, silver, copper, aluminum, zinc, nickel, iron, platinum, and/or combinations thereof. Optionally, the metal may be a thin layer. Optionally, the metal may be evaporated onto a support layer. Optionally, the metal layer may be a nanoparticle film. Optionally, the metal layer may have a thickness in the range between about 0.001 $\mu m$ to about 0.01 $\mu m$, between about 0.01 $\mu m$ to about 0.1 $\mu m$, between about 0.1 $\mu m$ to about 1 $\mu m$, between about 1 $\mu m$ to about 5 $\mu m$, between about 5 $\mu m$ to about 20 $\mu m$, between about 20 $\mu m$ to about 50 $\mu m$, between about 50 $\mu m$ to about 100 $\mu m$, or between about 100 $\mu m$ to about 500 $\mu m$.

According to some embodiments, a TENG device may include an adhesion layer. Optionally, the adhesion layer may lie between the metal layer and the support layer. Optionally, the adhesion layer may be a metal (e.g., tungsten, niobium, chrome, titanium, or oxides or combinations thereof). Optionally, the adhesion layer may have a thickness in the range between about 0.001 $\mu m$ to about 0.01 $\mu m$, between about 0.01 $\mu m$ to about 0.1 $\mu m$, between about 0.1 $\mu m$ to about 1 $\mu m$, between about 1 $\mu m$ to about 5 $\mu m$, or between about 5 $\mu m$ to about 20 $\mu m$.

According to some embodiments, a TENG device may include a support layer. Optionally, the support layer may be a polymer film, e.g., polyimide film (such as Kapton), etc. Optionally, the support layer may have a thickness in the range between about 0.01 $\mu m$ to about 0.1 $\mu m$, between about 0.1 $\mu m$ to about 1 $\mu m$, between about 1 $\mu m$ to about 5 $\mu m$, between about 5 $\mu m$ to about 20 $\mu m$, between about 20 $\mu m$ to about 50 $\mu m$, between about 50 $\mu m$ to about 100 $\mu m$, between about 100 $\mu m$ to about 500 $\mu m$, or between about 500 $\mu m$ to about 750 $\mu m$.

According to some embodiments, the TENG device and/or the electric wires may be coated with a biocompatible material. Optionally, the biocompatible material may encapsulate the device. Optionally, the encapsulating layer may prevent contact between the TENG device and the surrounding physiological environment, e.g., to prevent corrosion, toxicity, infection and/or rejection. Optionally, the biocompatible material may be selected from polydimethylsiloxane, fibrin glue, etc.

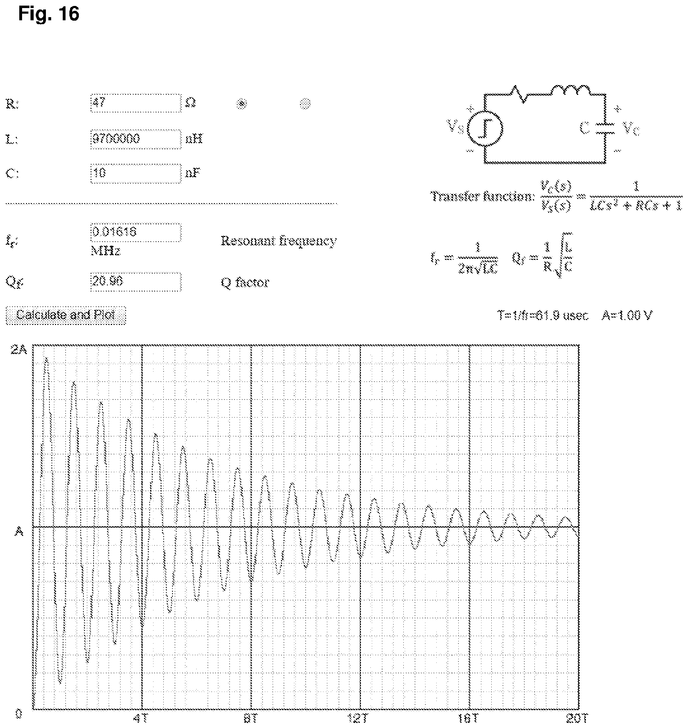

According to some embodiments, the TENG device may include circuitry that may change the electrical features of the electric pulse that was generated by the TENG e.g., **Fig. 16).** Optionally, the circuitry may change the pulse Optionally, PCB circuits and/or RCL circuits (e.g., resistor (R), an inductor (L), and a capacitor (C), connected in series or in parallel) may change the output response (e.g., the amplitude and/or frequency of the electrical signal). In some embodiments, utilizing such circuitry, may consequently change the neuronal response to the simulation.

According to some embodiments, there is provided a non-claimed method of restoring tactile sensation in a subject, may include one or more of the steps of:

subcutaneously implanting a sensory restoration device into the subject at a site of interest (e.g., fingertip), wherein the sensory restoration device comprises at least one nanogenerator unit and at least one electrode; connecting said at least one electrode to at least one functional sensory nerve of the subject; and stimulating said at least one functional sensory nerve by providing voltage/current thereto, wherein said current is generated by the nanogenerator unit and is proportional to an external stimulus exerted at a region external to the region in which the at least one nanogenerator unit is implanted.

According to some embodiments, there is provided a non-claimed method for at least partially

restoring a tactile sensation for a subject in need thereof, the method includes a step of providing a device comprising a nanogenerator device subcutaneously implanted and electrically connected to a functional nerve of the subject; wherein said device is configured for generating an electric signal by the nanogenerator in response to an external stimulus of a body region which corresponds to a subcutaneous location at which the at least one nanogenerator is located which is in direct or in-direct contact with said device, and convey the generated electric signal from nanogenerator via the electrode to a functional nerve associated with said electrode.

In some embodiments, there is provided a device as disclosed herein for use in at least partially restoring tactile sensation to a subject in need thereof.

According to some embodiments, there is provided a sensory restoration system which includes the sensory restoration device as disclosed herein and an external controller. In some embodiments, the controller may control one or more operating parameters of the device. In some embodiments, the controller may include a processor. In some embodiments, the controller may include a communication unit, such as, a wireless communication unit configured to allow interaction/communication with the device. In some embodiments, the controller may control the sensitivity of the sensory restoration device. In some embodiments, the controller may be configured to determine and/or provide feedback on the condition/operation of the sensory restoration device.

With reference to **Figs. 1A-B** show a schematic illustration of an exemplary TENG device, in accordance with some of the embodiments. **Fig. 1A** shows the implementation of a TENG device for restoring tactile sensation. TENG device 108 is implanted under the skin (*e.g.,* of a desensitized finger). Upon application of tactile pressure, the TENG device 108 generates an electrical signal, which is delivered using isolated cables 106 of stimulating cuff electrode 104 wrapped around the closest undamaged afferent nerve fiber 102, which can transduce a touch sensation signal 110 to the CNS. Top panel of Fig. 1B shows a photograph of an exemplary cuff electrode 112; and bottom panel of Fig. 1B shows a photograph of an exemplary device 114, showing TENG 118 portion and electrode cables 116.

**Fig. 2** is a schematic illustration of an TENG, which converts mechanical energy into electricity using the triboelectric effect, in accordance with some of the embodiments. According to some embodiments, the TENG device may be a self-powered device that implanted under the skin (e.g., at the fingertip) which transforms pressure (e.g., from touch) into voltage, which may be transduced to healthy sensory nerves via cuff electrodes, to excite peripheral nerves proportionally to the pressure that is applied on the device. For example, a positive triboelectric material 204 may be attached to a first electrode 202, and a negative triboelectric material 208 may be attached to a second electrode 210, with one or more spacers 206 in between. On applying pressure 214 to one or more sides of the TENG device, the positive 204 and negative 208 triboelectric material touch resulting in a charge in the first 204 and second 210 electrodes and a current is generated 212. On releasing 216 the pressure, the positive 204 and negative 208 triboelectric material no longer touch resulting in no charge in the first 204 and second 210 electrodes and no current is generated 212.

**Figs. 3A-B** show schematic illustrations of the working modes of the TENG, according to some embodiments. **Fig. 3A-** vertical contact separation mode; **Fig. 3B-** lateral sliding mode; **Fig. 3C-** single electrode mode, in accordance with some of the embodiments. For example, a positive triboelectric material 304 may be attached to a first electrode 302, and a negative triboelectric material 308 may be attached to a second electrode 310. The positive 304 and negative 308 triboelectric material may be brought into contact by exerting vertical pressure or laterally sliding them together resulting in a charge in the first 304 and second 310 electrodes and a current is generated 306. Optionally, only a single triboelectric material 308 may be attached to a second electrode 310, which may be brought into direct contact with a first electrode 302, without a second triboelectric material. Optionally, in such a case, the triboelectric material may be positively or negatively charged.

**Fig. 4** is an illustration of an exemplary TENG device implanted in a human finger, in accordance with some of the embodiments. Optionally, the TENG device may include an array of TENG, which may be activated by vertical pressure, lateral sliding, or a combination thereof. Optionally, the array may include a mixture of TENG which may be activated by vertical pressure or lateral sliding, (e.g., alternating, central, spiral, etc.). For example, vertical contact TENG 402 and lateral sliding TENG 404 in a checkerboard array connected to a nerve 408 by a cuff electrode 406. Optionally, the array may be composed of independent TENG sections and/or devices. In some embodiments, each independent TENG section and/or device may be different with respect of one or more of: size, shape, composition, charge, and the like. In some embodiments, the independent TENG section and/or device may be similar with respect of one or more of their properties, including, for example, size, shape, composition, charge, and the like. Optionally, each independent TENG section and/or device may have a unique property. Optionally, the number of independent TENG sections and/or devices may be between about 1 to 25, 1 to 5, between about 5 to 10, between about 10 to 25, between about 25 to 50, between about 50 to 100, between about 100 to 200, between about 200 to 500, or between about 500 to 1000. Each possibility is a separate embodiment. Optionally, the array may provide point discrimination.

**Fig. 5** is an exemplary manufacturing process for a TENG device, in accordance with some of the embodiments. For example, i. For both the positive and negative electrodes, a gold layer 502 may be evaporated by electron beam evaporation on to a support 504 (such as a film, e.g., Kapton). Optionally, an adhesion layer of titanium may first be evaporated onto the support to improve adhesion. ii. Then the gold-Kapton was soaked in an organic solution to improve adhesion of the dielectric material (e.g., mercaptohexadecanoic acid and/or ethanol to produce a self-assembled monolayer (SAM) of thiols) 505. iii. The Ka-Au base was held by vacuum, and dielectric materials (e.g., polydimethylsiloxane (PDMS), nylon (Ny), cellulose acetate butyrate (CAB), etc.) were spin-coated onto it by rotation at various speeds to produce a positive 508 or negative dielectric layer 508. Optionally, the coated Ka-Au strips were cured. Optionally, one or more conducting wires 511 may be attached to the gold electrode. iv. Optionally, a second layer of a support material may be added 510, to improve the structural resilience of the TENG. v. A spacer 512 (e.g., Very High Bond tape (VHB), etc.) of varying thicknesses (0, 125, 250, 500, 750, 1000, 1500, and 2000 $\mu$m) were placed on part of the dielectric layers 506 and 508. vi. The TENG was enclosed from both sides by a thin layer of VHB 514, and the edges were reinforced using Fibrin glue.

**Figs. 6A, Fig. 6B** and **Fig. 6C** are a side view, a perspective view and an expanded view, respectively, of a schematic illustration of an exemplary device design and final structure of a TENG device, in accordance with some of the embodiments. For example, the TENG device may include one or more support layer 602, one or more conducting layer 604, one or more dielectric layer 606, one or more spacer 608, one or more encapsulating layer 610, and one or more conducting wires 612.

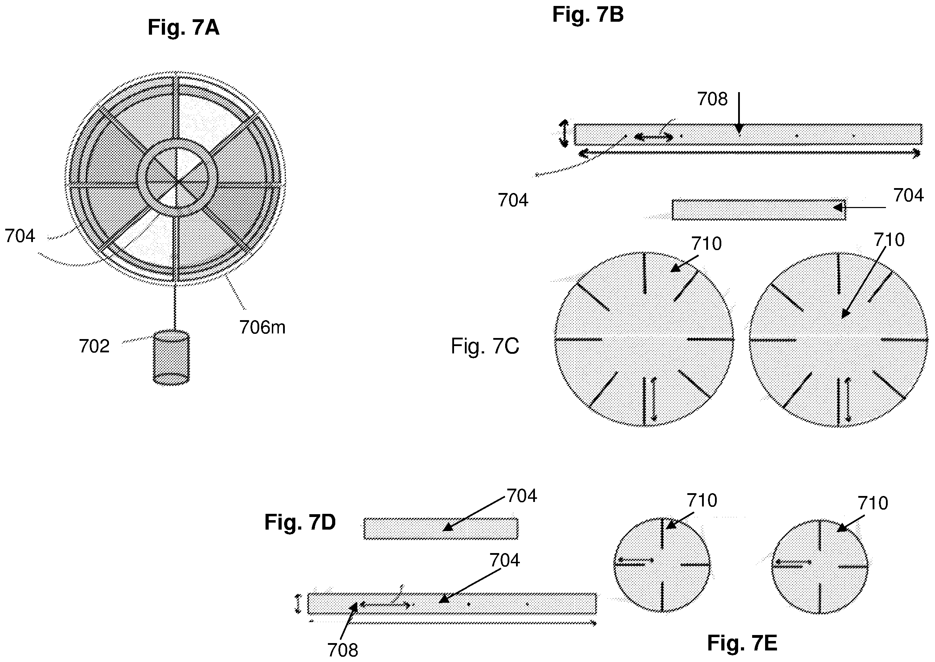

**Figs. 7A-E** shows a schematic illustration of an exemplary fixed design of a TENG array, **Fig. 7A** shows a top view of a TENG array connected to a cuff electrode 702 by one or more wires 706 and including one or more spacers 704. Optionally, the dielectric material (e.g., CAB) may cover all or part of a first layer (e.g., one or more plates). Optionally, a second material may cover all or part of a second layer (e.g., a second plate). Optionally, different segments may include the same or different dielectric materials. Optionally, different segments may include the same or different dielectric materials on a first layer and/or a second layer. **Fig. 7B** shows side view of one or more spacers 704 including multiple holes 708 in the spacer for wires to pass through, the spacers may be of various thicknesses and/or widths. **Fig. 7C** shows top view of first and second electrodes 710 of the TENG array. **Fig. 7D** shows a side view of one or more spacers 704, including holes 708 in at least one of the spacers 704 for wires to pass through, the distances between the holes may be uniform, and the spacer may be of various lengths. **Fig. 7E** shows a top view of first and second circles of the electrodes 710 of the TENG array.

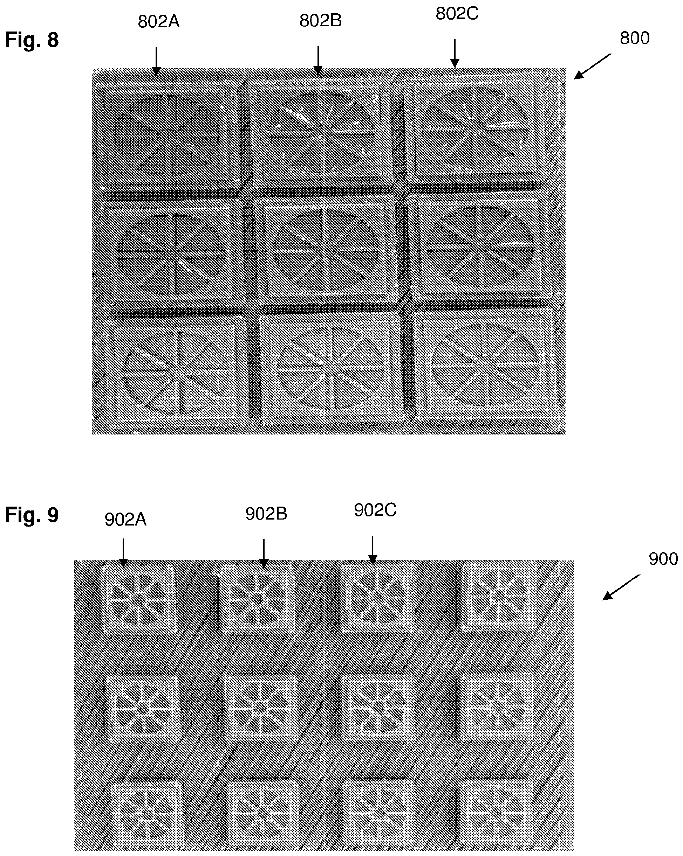

**Figs. 8** and **9** shows a top views of an exemplary templates prepared by 3D printing, in accordance with some of the embodiments. **Fig. 8** shows a 3D printed template 800, including individual TENG templates 802A-C. **Fig. 9** shows a 3D printed template 900, including individual TENG templates 902A-C. Optionally, the #D template may be of various sizes, and or depths, e.g., providing larger or smaller distances between the dielectric layers. Optionally, different heights between the dielectric layers may change the sensitivity of the TENG. For example, the template may act as a mold for one or more TENG arrays. Optionally, a biocompatible material e.g., polydimethylsiloxane, elastopolymer, etc. may be poured into the mold and provide the basis for one or more parts of a TENG. Optionally, the biocompatible layer may be coated by one or more additional layers, such as a conducting layer, adhesive layer, dielectric layer, etc. Optionally, a spacer may be included. Optionally, a supporting layer may not be required.

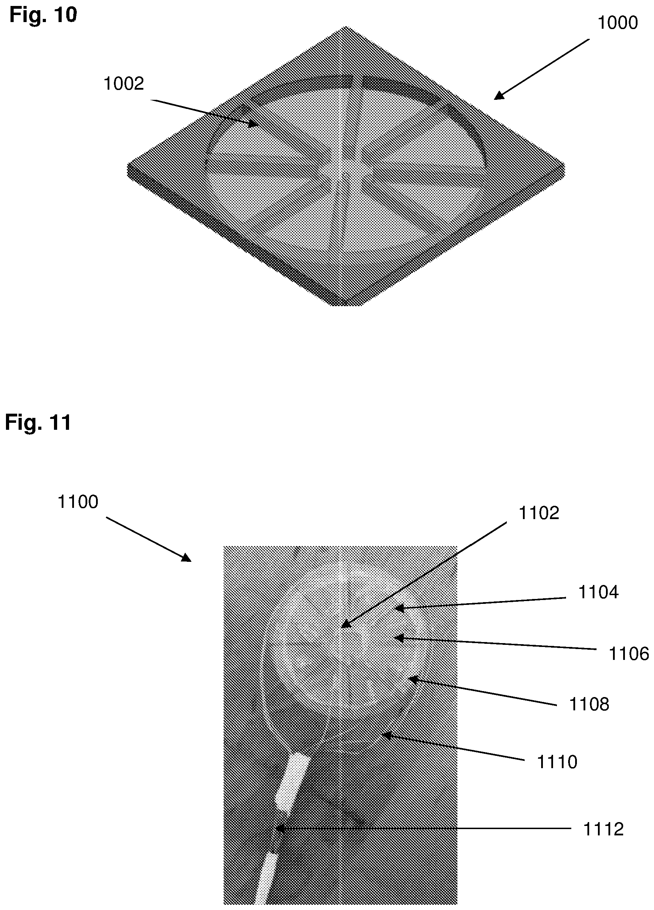

**Fig. 10** shows a perspective view of a schematic illustration of an exemplary TENG array template, in accordance with some of the embodiments. For example, the TENG array template 1000 may be produced from a 3D printed template. Optionally, radial supports may be included in the structure 1002. Optionally, the TENG may be configured to provide mechanical support. Optionally, the radial supports may be configured to act as spacers. Optionally, one or more templates and/or the TENG components produced therefrom may interlock. Optionally, one or more templates and/or the TENG components produced therefrom may be glued together.

Having thus described several embodiments for practicing the inventive device, its advantages and objectives can be easily understood. Variations from the description above may and can be made by one skilled in the art without departing from the scope of the invention.

Accordingly, this invention is not to be limited by the embodiments as described, which are given by way of example only and not by way of limitation.

Unless otherwise defined, all technical and/or scientific terms used herein have the same meaning as commonly understood by one of ordinary skill in the art to which the invention pertains. Although methods and materials similar or equivalent to those described herein may be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

The term "triboelectric effect" and "triboelectricity" are used interchangeably and are defined herein as the tendency of the material to gain or lose free electrons in a frictional or contact process.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the term "about" may be used to specify a value of a quantity or parameter (e.g., the length of an element) to within a continuous range of values in the neighborhood of (and including) a given (stated) value. According to some embodiments, "about" may specify the value of a parameter to be between 80 % and 120 % of the given value.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Unless otherwise defined, all technical and scientific terms used herein have the same meaning as commonly understood by one of ordinary skill in the art to which this disclosure pertains. In case of conflict, the patent specification, including definitions, governs. As used herein, the indefinite articles "a" and "an" mean "at least one" or "one or more" unless the context clearly dictates otherwise.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination or as suitable in any other described embodiment of the disclosure. No feature described in the context of an embodiment is to be considered an essential feature of that embodiment, unless explicitly specified as such.

Although steps of methods according to some embodiments may be described in a specific sequence, methods of the disclosure may include some or all of the described steps carried out in a different order. A method of the disclosure may include a few of the steps described or all of the steps described. No particular step in a disclosed method is to be considered an essential step of that method, unless explicitly specified as such.

Although the disclosure is described in conjunction with specific embodiments thereof, it is evident that numerous alternatives, modifications and variations that are apparent to those skilled in the art may exist. Accordingly, the disclosure embraces all such alternatives, modifications and variations that fall within the scope of the appended claims. It is to be understood that the disclosure is not necessarily limited in its application to the details of construction and the arrangement of the components and/or methods set forth herein. Other embodiments may be practiced, and an embodiment may be carried out in various ways.

The phraseology and terminology employed herein are for descriptive purpose and should not be regarded as limiting. Citation or identification of any reference in this application shall not be construed as an admission that such reference is available as prior art to the disclosure. Section headings are used herein to ease understanding of the specification and should not be construed as necessarily limiting.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art.

The following examples are presented to provide a more complete understanding of the invention. The specific techniques, conditions, materials, proportions and reported data set forth to illustrate the principles of the invention are exemplary and should not be construed as limiting the scope of the invention.

## EXAMPLES

### Example 1: Manufacturing of a TENG

**Fig. 11** shows an exemplary image of a top view of a TENG array, in accordance with some of the embodiments. For example, a TENG array 1100 including two oppositely charged electrodes 1106 comprising one or more layers (e.g., a support layer, a biocompatible layer, a conducting layer, an adhesive layer, a dielectric layer, etc.) may be separated by one or more spacers e.g., 1102, 1108. Each electrode 1106 may be connected to one or more wires 1110, which may carry a charge to a cuff electrode attached to an intact nerve (not shown). An exemplary detailed procedure follows.

### Gold Evaporation

Kapton strips (Ka) (Dupont, Delaware, USA) at a thickness of 13 $\mu$m or 125 $\mu$m served as the basis of the device structure, thus providing high flexibility and strength. A 5 nm adhesion layer of titanium (Ti) was evaporated by electron beam evaporation (VST, TFDS-870) on the Kapton strip. A 100 nm layer of gold (Au) was evaporated in the same way on top of the titanium layer serving as an electrode.

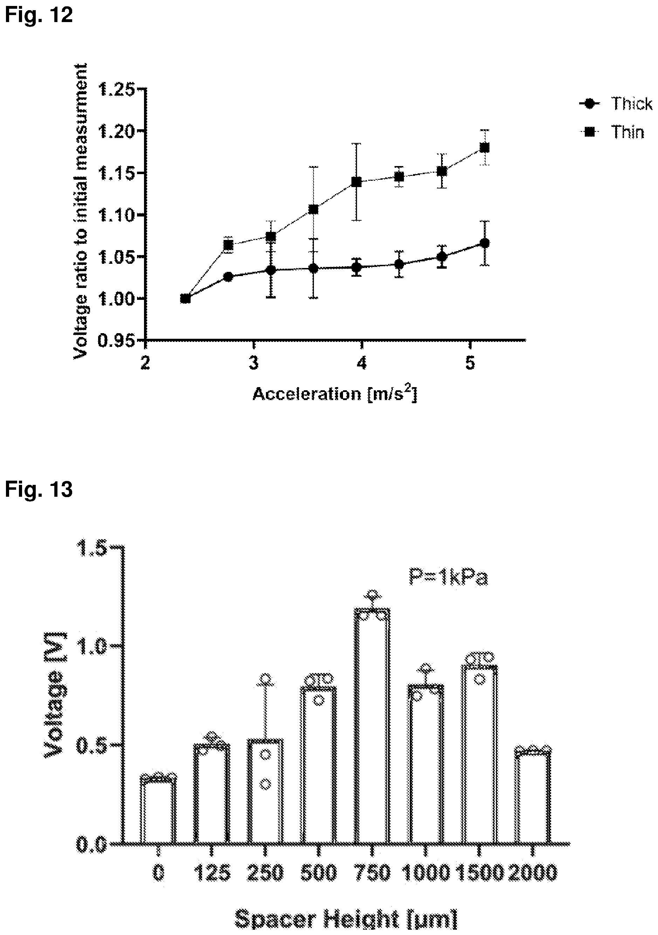

In the experiments for characterizing the effects of Kapton thickness on the TENG device's performance, the "thick" layer of Kapton was 125 $\mu$m thick (catalog number: 677-930-79) and the "thin" layer of Kapton was 13 $\mu$m thick (catalog number: 488-784-98) (e.g., Fig. 13).

### Adhesive Treatments

To improve attachment of the dielectric materials to the Gold layer, Ka-Au strips were soaked for 30 min in mercaptohexadecanoic acid (MHDA; Sigma-Aldrich, Rehovot, Israel) and diluted at a ratio of 1:100 in ethanol (Bio lab, Jerusalem, Israel), providing a self-assembled monolayer (SAM) of thiols for better adhesion with Nylon-6-6 (Sigma-Aldrich, Rehovot, Israel) and CAB (Sigma-Aldrich, Rehovot, Israel).

### Preparation of Dielectric Materials

PDMS (Sigma-Aldrich, Rehovot, Israel) was mixed at a ratio of 1:10 with curing agent (Sylgard 184, Sigma-Aldrich, Rehovot, Israel). Nylon-6-6 beads were dissolved in hexafluoro-2-propanol (HFIP; Sigma-Aldrich, Rehovot, Israel) at 60 mg/mL and sonicated at 45 °C for 30 min. CAB was dissolved in methyl isobutyl ketone (MIBK; Sigma-Aldrich, Rehovot, Israel) at 84 mg/mL. The solution was mixed until fully dissolved. CAB and Ny electrodes were soaked in MHDA solution (7.2 mg/mL in ethanol) to improve adhesion.

### Spin Coating

The Ka-Au base was held by vacuum, and dielectric materials (PDMS, Ny, CAB) were spin-coated onto it (Ni-Lo Scientific, Ottawa, Canada) at rotation velocities of 1000, 1800, and 1800 rpm for PDMS, CAB, and Ny, respectively. Each spin coat lasted 60 s, and final rotation speed was achieved in acceleration of 200 rounds/s$^2$. Large samples (when the TENG device is larger than 10 mm $\times$ 10 mm) were poured with 500 $\mu$L of the dielectric material in its liquid form and were used for initial characterization of the TENG device. Small samples (5 mm $\times$ 5 mm) were poured with 200 $\mu$L of the liquid dielectric coating.

### Curing

PDMS-coated Ka-Au strips were cured overnight (12 h) at 60 °C (ThermoFisher Scientific, Kiriyat Shemona, Israel). Ny and CAB Ka-Au strips were allowed to air-dry and attach overnight before device assembly and subsequent measurements.

**Wiring**

The exposed Au surface area was kept clear from the coating using elastic one-sided duct tape. Once the dielectric material was fully attached and cured, the duct tape was removed, and a copper wire with an exposed edge was connected using either fast-drying silver or gold paint (Ted Pella, Redding, California, United States) applied to the open Au area.

**Spacing**

Thin strips of Very High Bond tape (VHB; 3M, Teva Pharmaceuticals, Shoham, Israel) at varying thicknesses (0, 125, 250, 500, 750, 1000, 1500, and 2000 $\mu$m) were placed on top of the PDMS layer at each side of the upper surface area of the TENG device. The other side of the TENG device, containing the Ny/CAB layer, was inverted and placed directly above the PDMS layer (and separated at the sides by the VHB strips). Spacer optimization tests were conducted (e.g., **Fig. 13).**

**Encapsulation**

The TENG device was enclosed from both sides by a thin layer of 125 $\mu$m 3M VHB, and the edges were reinforced using Fibrin glue (Evicel - Omrix Biopharmaceuticals, J&J, Ness Ziona, Israel).

**Example 2: Response to Physiological Pressure**

Characterizing and evaluating the TENG device to verify that the device operates as expected in response to pressure within the physiological range, and specifically, that the relationship between the output voltage and the pressure applied fits the following equation:

$$V_{rel}(t) = \frac{V_{OC,0} - V_{OC}(t)}{V_{OC,0}} = \frac{S}{k \cdot d_0} \cdot p(t) \qquad \text{equation (1)}$$

where $V_{rel}$ is the relative change in voltage, Voc is the TENG device output voltage at a specific time point, $d_0$ is the maximal distance (*i.e.,* the size of the gap) between the two dielectric layers, $S$ is the surface area of the TENG device, k represents the TENG device's elasticity, and $p$ is the pressure applied.

For example, as can be seen in **Fig. 13,** the TENG device operates in the physiological range of pressure (as low as 1 kPa and high as 20 kPa), and the voltage-pressure relationship has high linear correlation to equation 1 ($R^2$ = 0.97). **Fig. 13** shows a graph showing average peak-to-peak output voltage from an encapsulated TENG with different spacer heights, upon application of 1 kPa pressure. N=3 each, in accordance with some of the embodiments. As can be seen, a spacer of 750 $\mu$m provided the highest voltage.

**Example 3: Sensitivity of the TENG**

The sensitivity of the TENG was dependent on the thickness of the Kapton layer supporting the electrode. Two different TENG devices were fabricated, one containing a "thick" (125 $\mu$m) layer of Kapton, and the other containing a "thin" (13 $\mu$m) layer. Each device's electrical output was then tested.

**Fig. 12** shows a graph showing the TENG response may be affected by the thickness of the supporting layer, in accordance with some embodiments. For example, the voltage vs. pressure for TENG devices (surface area 25 mm$^2$) made from either a "thick" layer of Kapton (125 $\mu$m) or a "thin" layer of Kapton (13 $\mu$m), in accordance with some of the embodiments.

The device that was fabricated using the "thin" layer of Kapton was more sensitive than the device with the "thick" layer of Kapton, and produced higher voltage for a given amount of pressure.

**Example 4: Durability of the TENG**

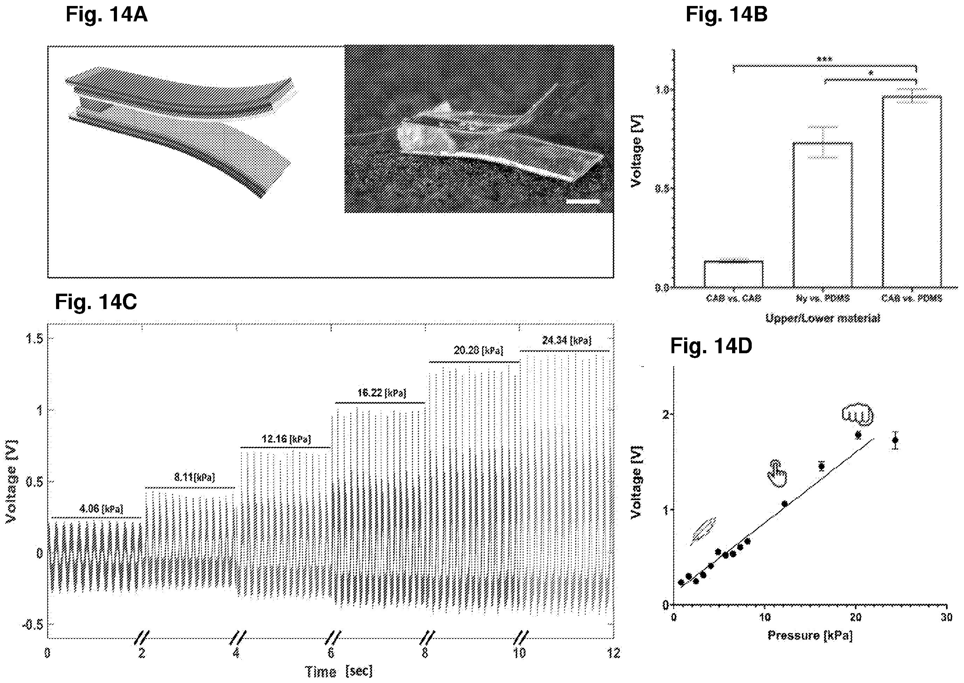

**Figs. 14A-D** show exemplary TENG characterization, in accordance with some of the embodiments. **Fig. 14A** shows a schematic of the TENG layers (left) and a photograph of an exemplary TENG (right) (scale bar: 2 mm). **Fig. 14B** shows mean peak-to-peak electrical output modulation of TENG (5 mm $\times$ 5 mm), as a function of the materials used for the friction layers. **Fig. 14C** shows an output performance of TENG (5 mm $\times$ 5 mm) for different levels of pressure applied. **Fig.**

**14D** shows TENG (5 mm $\times$ 5 mm) response to physiological pressure. A linear correlation ($R2 = 0.97$) between the average peak-to-peak output voltage and pressure applied to the device was observed.

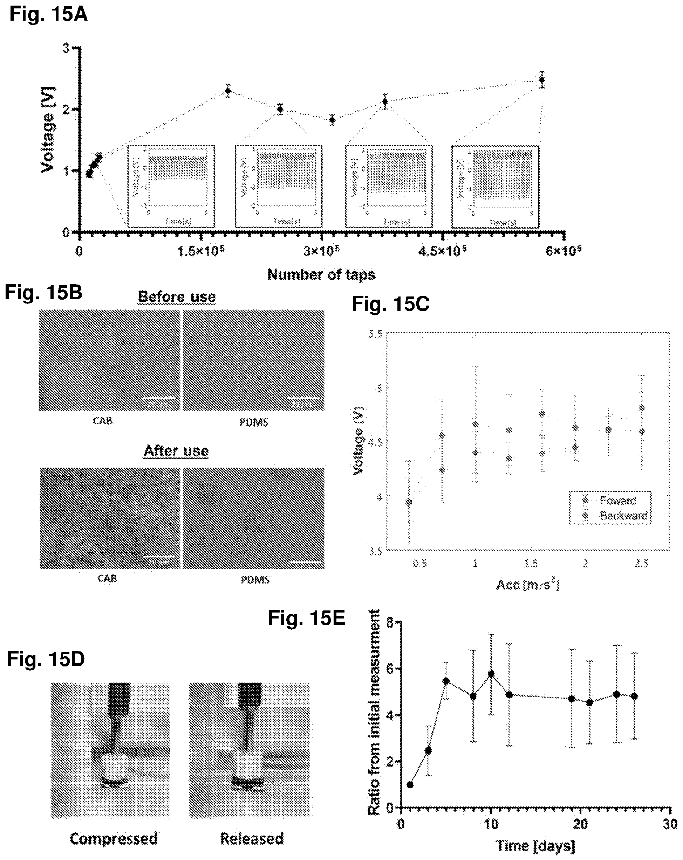

**Figs. 15A-E** show exemplary TENG characterization, in accordance with some of the embodiments. For example, **Fig. 15A.** Average peak-to-peak output voltage from TENG device. Insets in show raw data corresponding to 5 s intervals after specific time points. Pressure applied was 15 kPa at 4.5 Hz. **Fig. 15B** shows a SEM images of the TENG device (5 mm $\times$ 5 mm): top view of each layer of dielectric material (CAB or PDMS). The roughness of the surface of the dielectric materials changes for both PDMS and CAB, after >0.5 million taps of 15 kPa. **Fig. 15C** shows an average peak-to-peak output voltage of the TENG device in response to a set of increasing and decreasing accelerations (pressures) (labeled "forward" for increasing pressures and "backward" for decreasing pressures that immediately followed; $n$ = 3). **Fig. 15D** shows an example of TENG device compression. e. Average peak-to-peak output voltage divided by the initial peak-to-peak measurement ($n$ = 3). The pressure was applied by a motor moving in a sinusoidal motion at 1 Hz at peak acceleration of 3.95 m/s$^2$.

To evaluate the durability of the device, the inventors applied repeated pressure of 15 kPa at a frequency of 4.5 Hz over 36 h, resulting in more than 580,000 strong "finger taps" on the device (e.g., **Fig. 15A**). Over the course of the first 11,000 taps, the output voltage from the TENG increased to 128% of its starting value. After approximately 170,000 taps, the final output voltage arrived at saturation ($2.487 \pm 0.133$ V), corresponding to 261% of the initial value. In an effort to identify the cause of this increase in voltage over time, the inventors examined scanning electron microscope (SEM) images of the surface area of the dielectric materials; the images had been recorded over the course of the experiment (e.g., **Fig. 15B**). It was observed that the surface of the dielectric materials (mainly the CAB) became rougher over time. As a TENG's triboelectric effect is highly dependent on its surface area (equation (1)), the roughening of the surface apparently increased the output voltage for a given pressure (e.g., **Fig. 15A** and **Fig. 15B**).

The robustness of the TENG device in the presence of multiple forces over time was evaluated (e.g., **Fig. 15C** and **Fig. 15D**). During force application, it was verified that the two layers touched each other and went back to their original state (e.g., **Fig. 15B**). The results show that the dynamic range of the TENG device is not significantly affected by the application of high pressure, which is a crucial condition for in vivo use.

### Example 5: Resistance to Biological Conditions

Exposure to biological conditions such as moisture, body temperature, and salinity might cause degradation via corrosion, swelling, and hydrolysis. To evaluate how the TENG device might respond to such exposure over time, and to validate the long-term stability of the device, the following procedure was performed. Over a period of 26 days, TENG device devices were kept in PBS solution in an incubator at 37 °C - an environment simulating biological conditions. The device's output voltage in response to peak acceleration of 3.95 m/s$^2$ was measured 3 times a week for 30 min. Tt was observed that after a stabilization period of ~5 days, the output voltage remained relatively stable as compared to the initial measurement (e.g., **Fig. 15E**). Notably, this pattern resembles the pattern observed in our initial durability test, in the absence of biological conditions (e.g., **Fig. 15A).**

### Example 6: Change in the electrical features of the electric pulse

**Fig. 16** shows a graph showing an exemplary change in the electrical features of the electric pulse generated by the TENG, and exemplary circuitry of the array, in accordance with some of the embodiments. For example, a specific RCL circuit may change the response of the electric output of the TENG, such as, by increasing the frequency of the electric output.

### Example 7: TENG device Activates Sensory Neurons in Vitro

*In vitro* proof-of-principle by characterizing the TENG device's capacity to activate sensory neurons and, specifically, mouse dorsal root ganglia (DRGs) was performed. A multielectrode array (MEA) platform that could be integrated with the TENG device (TENG device surface area: 25 mm$^2$ [5 mm $\times$ 5 mm]) was developed. The MEA was used both to measure cells' electrical activity and to generate potential that stimulates the cells.

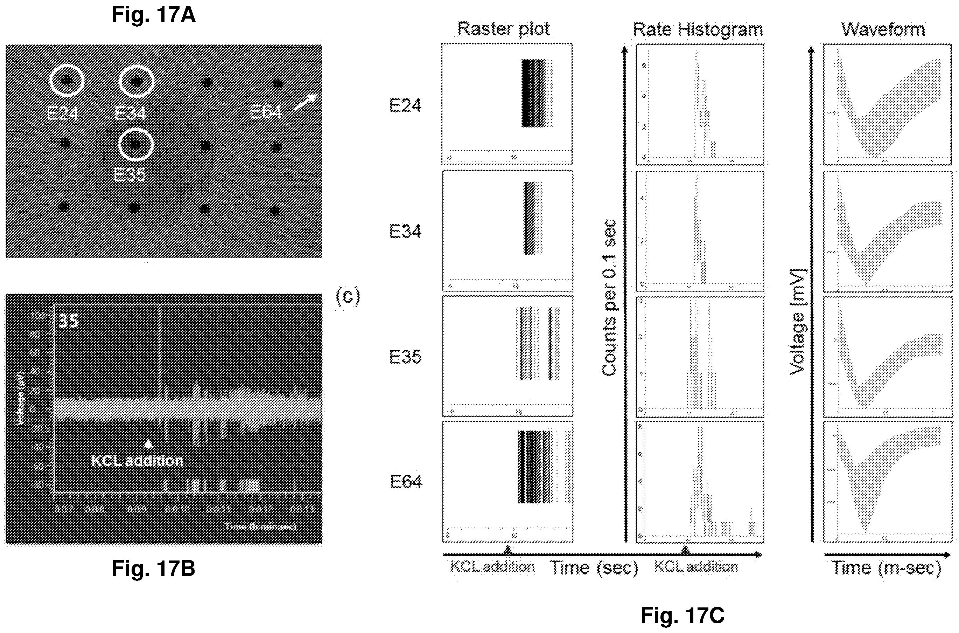

DRGs are known to exhibit limited spontaneous electrophysiological activity in vitro. MEA measurements of the DRGs' baseline activity did not show significant spontaneous electrical activity (e.g., **Figs. 17A-C).** To verify that the cells were capable of electrical activity, the inventors exposed them to KCl (in line with prior studies) and observed that, as expected, electrical activity increased following KCl addition.

**Figs. 17A-C** show *in vitro* testing of an exemplary TENG, in accordance with some of the embodiments. For example, **Fig. 17A** shows a $\times$10 image of an exemplary DRG on MEA culture at DIV 5, with complete axonal coverage. Analyzed electrodes are marked in white circles. **Fig. 17B** shows Raw MEA data, with the threshold for spike definition marked as a horizontal line, and vertical lines generating the raster plot of activity. KCl was added to reach 50 mM concentration at $t$ = 10 s. **Fig. 17C** shows a Data analysis for 4 representative electrodes: Raster plot, rate histogram of spikes per 0.1 seconds, and average waveform shape for each spike.

Preliminary experiments indicated that the DRG response to stimulation (electrical potential) generated by the TENG device improves when the cells are first primed to be electrically active, rather than stimulated from their baseline (inactive) state (data not shown). Because KCl exposure kills the cells, the capacity of the MEA's stimulation system to prime the DRGs in this manner was evaluated. It was observed that, indeed, exposure to MEA stimulation elicited a significant increase in the DRGs' spontaneous electrical activity (e.g., **Fig. 17B).**

The DRGs' capacity to respond differentially to different levels of electrical potential was evaluated. This step was aimed at supporting a basic assumption underlying the TENG device concept, which is that neurons can sense different levels of electrical potential, generated by different levels of tactile pressure on the implanted TENG device (and ultimately transmit this information as tactile sensory information to the brain). In this experiment, the inventors exposed the (electrically primed) DRGs to external electric potential, generated by the MEA itself, and proportional to the potential that was expected to be generated by the TENG device. Quantification of the neuronal electrical activity showed that, indeed, the DRGs' electrical activity was more intensive in the presence of higher levels of voltage used for DRG stimulation.

The final step in the in vitro testing process was to expose (electrically primed) DRGs to direct stimulation from the TENG device. As shown in **Fig. 17E,** TENG device stimulation elicited electrical activity in the DRGs.

## Example 8: Surgical Procedures: Sensory Nerve Transection and TENG device Implantation

12-week-old female Wistar rats ($n$ = 9) were divided into 3 groups: "control" ($n$ = 3), in which no procedure was done; "amputee" ($n$ = 3), in which a segment of the left distal tibial nerve was removed; and "TENG device" ($n$ = 3), in which a segment of the left distal tibial nerve was removed, and a TENG device (surface area: 8 mm $\times$ 3 mm) was implanted at the left hindfoot and connected with cuff electrodes to the terminal part of the remaining portion of the left distal tibial nerve (e.g., **Fig. 18).** The implanted device was encapsulated and sealed with biocompatible materials, to prevent inflammation. After the rats recuperated from surgery, the inventors monitored their movement (measured according to the amount of time rats spent on their hindfeet over the course of 1 min) and observed no significant differences between the groups, suggesting that all animals were well and healthy, and their motor capability was not affected.

For surgery, rats in the amputee and TENG device groups were anesthetized, and then an incision was made in the lateral part of the left posterior foot (as described earlier), and a segment of the medial and lateral tibial nerves was removed.

For rats in the TENG device group, the TENG device was placed subcutaneously in the central part of the rat's paw and then attached to the terminal part of the transected tibial nerve using a cuff electrode (2 Micro Cuff Tunnel 0,0000 1.556,70 200/Pt-Ir/2 mm long/0.5 mm C2C/0.2 $\times$ 0.5 mm O/Cable 30 cm entry lateral - weld tube 001; CorTec, Saint Paul, Minnesota, United States). A 9-0 nylon suture was used to secure the positioning and the attachment of the cuff to the nerve.

The surgical incision was sutured with a 5-0 nylon suture, and the rats were treated with antibiotics and painkillers (Rymadil; Vetmarket, Shoham, Israel). After the surgery, the rats recovered for 10 days before the von Frey tests.

## Example 9: Sensation Assessment: von Frey Test

A von Frey test was used to measure the rats' sensation. In this setup, increasing force is applied to the rat's paw from below, and once the rat senses the force, it lifts its paw. Rats with functional tactile sensation respond to low amounts of force, whereas rats lacking tactile sensation respond only to much higher levels of force. It should be noted that even rats in which the distal tibial nerve has been severed would eventually respond, as high levels of force can move the entire leg, and then the rat will notice the force that has been applied.

The von Frey apparatus (Ugo Basile, Italy) consists of an elevated horizontal wire mesh stand. The rat stands on top of the wire mesh, inside a plexiglass enclosure with an open bottom. Pressure is applied to the rat's paw from below, using a tip. When the rat lifts its foot, indicating that it has sensed the pressure applied, the maximal force applied is automatically recorded by an electronic device. Each rat underwent the von Frey test once every 3 or 4 days, over the course of 19 days, as noted above. Each animal was subjected to 5 measurements in each hind leg, on each measurement day.

Since different animals were likely to have different thresholds for sensation, the inventors tested both hindfeet (treated and untreated) as a within-subjects control, in addition to using a control group. Comparing measurements in the left hindfoot (*i.e.,* the treated hindfoot in the amputee and TENG device groups), it was observed that the control group responded to a low level of force (2.69 $\pm$ 0.12g), significantly lower than that required to elicit a response from the amputee group, which responded only to high levels of force (14.12 $\pm$ 2.53g). Rats in the TENG device group, in turn, responded to a much lower amount of force compared with the amputee group (3.99 $\pm$ 3.54g, $p$-value = 0.0099); this level was similar to the amount of force required for the control group.

**Figs. 18A-E** show *in vivo* testing of an exemplary TENG, in accordance with some of the embodiments. For example, **Fig. 18A** shows a schematic illustration based on anatomical dissection of the nerves of a Wistar rat's hindfoot. **Fig. 18B** shows images of surgical implantation of TENG device (8 mm × 3 mm) in a rat's left hindfoot, and post-operative recovery (scale = 1 cm). **Fig. 18C** shows percentage of time spent on hind paws for each group, over the course of 1 min. **Fig. 18D** shows an exemplary von Frey test setup. Arrow points to the tip of the device. **Fig. 18E.** Difference in average required peak force between the right and left hind paws in the von Frey test, measured for three experimental groups: control, amputee, TENG device (*P*-values: \*\**p* = 0.0099, \**p* = 0.0242).

The characteristics of the TENG device sample were similar to those of the amputee sample, suggesting that both groups experienced similar nerve damage (due to the removal of part of the nerve). The fact that TENG device rats experienced nerve damage yet showed sensory capabilities similar to those of the control rats lends support to our assumptions regarding the TENG device's mode of operation: namely, the cuff electrode attached to the TENG device bypasses the damaged area of the nerve and relays signals to the (healthy) nerve to which it is attached, enabling tactile sensation capabilities to be (at least to some extent) restored.

**Claims**

1. An implantable device for at least partially restoring tactile sensation to a subject in need thereof, the device comprising:

   at least one nanogenerator unit (108, 118) configured to be positioned subcutaneously and to produce an electric signal upon exertion of an external stimulus; and
   at least one electrode (104, 112) configured to be connected to at least one functional sensory nerve of the subject;
   such that the electric signal produced by the at least one nanogenerator unit (108, 118) in response to the external stimulus is conveyed to the at least one functional sensory nerve (102) of the subject, via the at least one electrode (104, 112) ;
   **characterized in that** said nanogenerator unit (108, 118) is further coated with a heat sensitive material, thereby at least partially restoring the tactile sensation to the subject.

2. The sensory restoration device according to claim 1, wherein the external stimulus comprises: pressure, friction, traction, shear force and/or temperature change.

3. The sensory restoration device according to any one of claims 1-2, wherein said nanogenerator unit (108, 118) is connected to a single nerve.

4. The sensory restoration device according to any one of claims 1-3, comprising a plurality of nanogenerator units (108, 118) and wherein the plurality of nanogenerator units are adapted to be connected to a single nerve.

5. The sensory restoration device according to any one of claims 1-4, wherein the nanogenerator unit is selected from the group consisting of a piezoelectric nanogenerator, and a triboelectric nanogenerator.

6. The sensory restoration device according to claim 5, wherein the piezoelectric nanogenerator: (a) has a wurtzite structure or a perovskite structure; or (b) is a pressure sensor.

7. The sensory restoration device according to claim 5, wherein the triboelectric nanogenerator, TENG, (118) is configured to convert pressure, friction and/or traction force applied to said TENG (118) into an electric signal that is supplied to said at least one sensory nerve.

8. The sensory restoration device according to claim 7, further comprising a circuitry which is configured to change the electrical features of an electric pulse generated by the TENG (118).

9. The sensory restoration device according to any one of claims 1-8, wherein said nanogenerator is self-powered.

10. A system comprising the device of any one of claims 1-9 and an external controller configured to control one or more operating parameters of the device.

11. The system according to claim 10, wherein the controller is wirelessly associated with the device.

**12.** The system according to any one of claims 10-11, wherein the controller is configured to control the sensitivity of the sensory restoration device.

**13.** The system according to any one of claims 10-12, wherein the controller is configured to determine and/or provide feedback on the condition/operation of the sensory restoration device.


**Patentansprüche**

**1.** Implantierbare Vorrichtung zur mindestens teilweisen Wiederherstellung des Tastsinns bei einem Subjekt, der dessen bedarf, wobei die Vorrichtung umfasst:

mindestens eine Nanogeneratoreinheit (108, 118), die konfiguriert ist, um subkutan positioniert zu werden und bei Ausüben eines externen Reizes ein elektrisches Signal zu erzeugen; und
mindestens eine Elektrode (104, 112), die konfiguriert ist, um mit mindestens einem funktionsfähigen Sinnesnerv des Subjekts verbunden zu werden;
sodass das elektrische Signal, das von der mindestens einen Nanogeneratoreinheit (108, 118) als Reaktion auf den externen Reiz erzeugt wird, über die mindestens eine Elektrode (104, 112) an den mindestens einen funktionsfähigen Sinnesnerv (102) des Subjekts vermittelt wird;
**dadurch gekennzeichnet, dass** die Nanogeneratoreinheit (108, 118) weiter mit einem wärmeempfindlichen Material beschichtet ist, wodurch dem Subjekt mindestens teilweise der Tastsinn wiederhergestellt wird.

**2.** Vorrichtung zur Sinneswiederherstellung nach Anspruch 1, wobei der externe Reiz umfasst: Druck, Reibung, Zug, Scherkraft und/oder Temperaturänderung.

**3.** Vorrichtung zur Sinneswiederherstellung nach einem der Ansprüche 1-2, wobei die Nanogeneratoreinheit (108, 118) mit einem einzigen Nerv verbunden ist.

**4.** Vorrichtung zur Sinneswiederherstellung nach einem der Ansprüche 1-3, umfassend eine Vielzahl von Nanogeneratoreinheiten (108, 118), und wobei die Vielzahl von Nanogeneratoreinheiten angepasst sind, um mit einem einzigen Nerv verbunden zu werden.

**5.** Vorrichtung zur Sinneswiederherstellung nach einem der Ansprüche 1-4, wobei die Nanogeneratoreinheit aus der Gruppe bestehend aus einem piezoelektrischen Nanogenerator und einem triboelektrischen Nanogenerator ausgewählt ist.

**6.** Vorrichtung zur Sinneswiederherstellung nach Anspruch 5, wobei der piezoelektrische Nanogenerator: (a) eine Wurtzitstruktur oder Perowskitstruktur aufweist; oder (b) ein Drucksensor ist.

**7.** Vorrichtung zur Sinneswiederherstellung nach Anspruch 5, wobei der triboelektrische Nanogenerator, TENG, (118) konfiguriert ist, um Druck, Reibung und/oder Zugkraft, ausgeübt auf den TENG (118), in ein elektrisches Signal umzuwandeln, das dem mindestens einen Sinnesnerv zugeführt wird.

**8.** Vorrichtung zur Sinneswiederherstellung nach Anspruch 7, weiter umfassend eine Schaltung, die konfiguriert ist, um die elektrischen Merkmale eines vom TENG (118) erzeugten elektrischen Impulses zu ändern.

**9.** Vorrichtung zur Sinneswiederherstellung nach einem der Ansprüche 1-8, wobei der Nanogenerator selbstversorgend ist.

**10.** System, umfassend die Vorrichtung nach einem der Ansprüche 1-9 und eine externe Steuereinheit, die konfiguriert ist, um einen oder mehrere Betriebsparameter der Vorrichtung zu steuern.

**11.** System nach Anspruch 10, wobei die Steuereinheit drahtlos mit der Vorrichtung verknüpft ist.

**12.** System nach einem der Ansprüche 10-11, wobei die Steuereinheit konfiguriert ist, um die Empfindlichkeit der Vorrichtung zur Sinneswiederherstellung zu steuern.

**13.** System nach einem der Ansprüche 10-12, wobei die Steuereinheit konfiguriert ist, um den Zustand/Betrieb der

**EP 4 408 517 B1**

Vorrichtung zur Sinneswiederherstellung zu bestimmen und/oder eine Rückmeldung darüber bereitzustellen.

**Revendications**

1. Dispositif implantable destiné à restaurer, au moins partiellement, la sensation tactile chez un sujet qui en a besoin, ce dispositif comprenant :

   au moins une unité de nanogénérateur (108, 118) configurée pour être positionnée sous la peau et pour produire un signal électrique sous l'effet d'un stimulus externe ; et
   au moins une électrode (104, 112) configurée pour être connectée à au moins un nerf sensoriel fonctionnel du sujet ;
   de sorte que le signal électrique produit par l'au moins une unité de nanogénérateur (108, 118) en réponse au stimulus externe soit transmis à au moins un nerf sensoriel fonctionnel (102) du sujet, via l'au moins une électrode (104, 112) ;
   **caractérisé en ce que** ladite unité de nanogénérateur (108, 118) est en outre recouverte d'un matériau thermosensible, restaurant ainsi au moins partiellement la sensation tactile au sujet.

2. Dispositif de restauration sensorielle selon la revendication 1, dans lequel le stimulus externe comprend : pression, frottement, traction, force de cisaillement et/ou changement de température.

3. Dispositif de restauration sensorielle selon l'une quelconque des revendications 1-2, dans lequel ladite unité de nanogénérateur (108, 118) est connectée à un seul nerf.

4. Dispositif de restauration sensorielle selon l'une quelconque des revendications 1-3, comprenant une pluralité d'unités de nanogénérateur (108, 118) et dans lequel la pluralité d'unités de nanogénérateur est adaptée pour être connectée à un seul nerf.

5. Dispositif de restauration sensorielle selon l'une quelconque des revendications 1-4, dans lequel l'unité de nanogénérateur est choisie parmi le groupe constitué d'un nanogénérateur piézoélectrique et d'un nanogénérateur triboélectrique.

6. Dispositif de restauration sensorielle selon la revendication 5, dans lequel le nanogénérateur piézoélectrique : (a) présente une structure de wurtzite ou une structure de pérovskite ; ou (b) est un capteur de pression.

7. Dispositif de restauration sensorielle selon la revendication 5, dans lequel le nanogénérateur triboélectrique (TENG) (118) est configuré pour convertir la pression, le frottement et/ou la force de traction appliquée audit TENG (118) en un signal électrique qui est fourni audit au moins un nerf sensoriel.

8. Dispositif de restauration sensorielle selon la revendication 7, comprenant en outre un circuit configuré pour modifier les caractéristiques électriques d'une impulsion électrique générée par le TENG (118).

9. Dispositif de restauration sensorielle selon l'une quelconque des revendications 1-8, dans lequel ledit nanogénérateur est auto-alimenté.

10. Système comprenant le dispositif selon l'une quelconque des revendications 1-9 et un dispositif de commande externe configuré pour commander un ou plusieurs paramètres de fonctionnement du dispositif.

11. Système selon la revendication 10, dans lequel le dispositif de commande est associé sans fil au dispositif.

12. Système selon l'une quelconque des revendications 10-11, dans lequel le dispositif de commande est configuré pour commander la sensibilité du dispositif de restauration sensorielle.

13. Système selon l'une quelconque des revendications 10-12, dans lequel le dispositif de commande est configuré pour déterminer et/ou fournir des commentaires sur l'état/le fonctionnement du dispositif de restauration sensorielle.

**Fig. 1A**

102 104 106 108

110

112

116 114 118

**Fig. 1B**

**Fig. 2**

202
204
206
208
210

212

214 Press

216 Release

Fig. 3A

302
304
308
310
A
306

Fig. 3B

302
304
308
310
A
306

Fig. 3C

304
308
310
A
306

Fig. 4

402
404
406
408

**Fig. 5**

## Fig. 6A

604    610    608

612

## Fig. 6B

610

606    610    608
604    602

## Fig. 6C

602
604
606
608
610
612

**Fig. 7A**

**Fig. 7B**

704

704

704

708

710

710

Fig. 7C

704

Fig. 7D

708

704

710

710

Fig. 7E

702

706m

**Fig. 8**

802A    802B    802C    800

**Fig. 9**

902A    902B    902C    900

**Fig. 10**

1000

1002

**Fig. 11**

1100

1102

1104

1106

1108

1110

1112

**Fig. 12**

**Fig. 13**

**Fig. 14A**

**Fig. 14B**

**Fig. 14C**

**Fig. 14D**

**Fig. 15A**

**Fig. 15B**

Before use

CAB          PDMS

After use

CAB          PDMS

**Fig. 15C**

**Fig. 15D**

Compressed          Released

**Fig. 15E**

# Fig. 16

R:         [47        ] Ω          ⊙          ○

L:         [9700000   ] nH

C:         [10        ] nF

---

$f_r$:      [0.01616   ]
           MHz                    Resonant frequency

$Q_f$:      [20.96     ]           Q factor

[ Calculate and Plot ]

Transfer function: $\dfrac{V_C(s)}{V_S(s)} = \dfrac{1}{LCs^2 + RCs + 1}$

$f_r = \dfrac{1}{2\pi\sqrt{LC}}$    $Q_f = \dfrac{1}{R}\sqrt{\dfrac{L}{C}}$

T=1/fr=61.9 usec    A=1.00 V

**Fig. 17A**

**Fig. 17B**

**Fig. 17C**

**Fig. 18A**

**Fig. 18B**

Surgery      1 Week      2 Weeks      3 Weeks

**Fig. 18C**            **Fig. 18D**            **Fig. 18E**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150076964 A1, MADANI

- CN 111408046 A

**Non-patent literature cited in the description**

- **SHLOMY IFTACH et al.** Restoring Tactile Sensation Using a Triboelectric Nanogenerator. *ACS NANO*, 17 June 2021, vol. 15 (7), 11087-11098